# EUROPÄISCHE PATENTSCHRIFT

(11) **EP 3 693 258 B1**
(45) Veröffentlichungstag und Bekanntmachung des Hinweises auf die Patenterteilung: **27.04.2022**
(21) Anmeldenummer: 20156244.4
(22) Anmeldetag: 07.02.2020
(51) Int. Cl.: B62J 45/40, G01P 5/16

(54) **ZWEIRADKOMPONENTE MIT EINER MESSEINRICHTUNG**
BICYCLE COMPONENT WITH A MEASURING DEVICE
COMPOSANT À DEUX ROUES DOTÉ D'UN DISPOSITIF DE MESURE

(30) Priorität: 09.02.2019 DE 102019103232
(43) Veröffentlichungstag der Anmeldung: 12.08.2020
(73) Patentinhaber: DT Swiss AG, 2504 Biel (CH)
(72) Erfinder: Walthert, Martin, CH-3270 Aarberg (CH); Hugentobler, Simon, CH-3097 Liebefeld (CH); Ballard, Jean-Paul Victor, CH-8800 Thalwil (CH); Mullarkey, Seamus, CH-8632 Tann (CH); Jonasson, Jonas Gretar, CH-8800 Thalwil (CH)
(74) Vertreter: BSB Patentanwälte Schütte & Engelen Partnerschaft mbB

(56) Entgegenhaltungen:
- WO-A1-2017/103878
- WO-A1-2017/197524
- US-A- 4 987 542
- US-B1- 7 716 980
- Ray Maker: "Hands-on: New $299 AeroPod Aerodynamic Sensor | DC Rainmaker", , 18. April 2018 (2018-04-18), XP055698915, Gefunden im Internet: URL:https://www.dcrainmaker.com/2018/04/ha nds-on-new-299-aeropod-aerodynamic-sensor. html [gefunden am 2020-05-27]

## Beschreibung

Die vorliegende Erfindung betrifft eine Zweiradkomponente mit einer Messeinrichtung mit wenigstens einer Messsonde und wenigstens einem Luftdrucksensor, um ein Maß für einen Umgebungsdruck an einem Fahrrad zu erfassen, in dem die Zweiradkomponente angebracht ist. Beispielsweise kann aus einem Luftdrucksignal eines Luftdrucksensors die aktuelle Höhe des Zweirads berechnet werden bzw. es kann insbesondere eine Höhenänderung während der Fahrt erfasst werden. Derartige Messungen können für unterschiedliche Zwecke eingesetzt werden. Beispielsweise ist es möglich, die Daten für eine spätere Auswertung zu speichern. Mit diesen und weiteren Messdaten kann aber auch ein Luftwiderstand des Zweirads inklusive der sich darauf befindlichen Fahrers beim Fahren ermittelt werden.

Um ein Verstopfen des Strömungskanals einer Pitot-Sonde bei Flugzeugen zu verhindern sind, werden in der US 7,716,980 B1 verschiedene geometrische und Materialkonfigurationen vorgeschlagen. Aus der WO 2017/297524 A1 sind eine Methode und ein Apparat bekannt geworden, um den Luftwiderstand eines Objekts wie eines Fahrrads zu bestimmen. WO 2017/197524 A1 offenbart den Oberbegriff des Anspruchs 1.

Auch am Fahrrad angebrachte Zweiradkomponenten sind unterschiedlichsten Witterungsbedingungen ausgesetzt. So kann es auch bei anfangs gutem Wetter während einer Tour oder eines Rennens zu plötzlichen Niederschlag kommen. Dann müssen die an einem Fahrrad eingesetzten Zweiradkomponenten entweder unempfindlich gegenüber Feuchtigkeit sein oder so aufgebaut sein, dass ein Regenschauer die eingesetzten Zweiradkomponenten nicht beschädigt. Ein wesentlicher Aspekt ist auch der Energiebedarf. So kann bei einem Fahrrad die Messapparatur nicht erst aufwendig temperiert und gekühlt oder geheizt werden, um unter allen Umgebungsbedingungen gleiche Resultate zu liefern. Es ist deshalb die Aufgabe der vorliegenden Erfindung, eine Zweiradkomponente mit einer Messeinrichtung zur Verfügung zu stellen, welche weitestgehend unempfindlich gegenüber Feuchtigkeitseinflüssen beim Fahren oder auch im Stillstand ist. Insbesondere soll die Messeinrichtung auch umempfindlich gegen andere Verschmutzungen sein.

Diese Aufgabe wird gelöst durch eine Zweiradkomponente mit den Merkmalen des Anspruchs 1. Bevorzugte Weiterbildungen der erfindungsgemäßen Zweiradkomponente sind Gegenstand der Unteransprüche. Weitere Vorteile und Merkmale der vorliegenden Erfindung ergeben sich aus der allgemeinen Beschreibung und der Beschreibung der Ausführungsbeispiele.

Eine erfindungsgemäße Zweiradkomponente umfasst insbesondere ein Gehäuse und wenigstens eine Messeinrichtung und wenigstens eine mit dem Gehäuse verbundene Messsonde. Die Messsonde umfasst wenigstens einen besonders bevorzugt ungeheizten Sondenkörper, der wenigstens eine äußere Öffnung (oder auch mehrere äußere Öffnungen insbesondere am Sondenkörper) aufweist, wobei die äußere Öffnung durch eine insbesondere interne Luftführung mit wenigstens einem oder auch mehreren von der äußeren Öffnung entfernt angeordneten Luftluftdrucksensor bzw. Luftdrucksensoren verbunden ist. Die (interne) Luftführung weist wenigstens zwei oder auch mehr Luftkanäle auf und umfasst wenigstens eine insbesondere innere Kammer, die insbesondere durch einen oder mehrere Ein- und Ausgänge mit wenigstens zwei Luftkanälen verbunden ist. Dabei ist einer der Luftkanäle als Zufuhrkanal ausgebildet und beginnt an der äußeren Öffnung (des am Sondenkörpers). Ein anderer Luftkanal dient als Sensorkanal und verbindet die innere Kammer mit dem Luftdrucksensor. Die Verbindung kann dabei mittelbar oder unmittelbar sein. Wenigstens einer der Luftkanäle weist oder wenigstens zwei Luftkanäle oder alle Luftkanäle weisen einen minimalen lichten Durchmesser kleiner 2,5 mm auf.

Die erfindungsgemäße Zweiradkomponente hat viele Vorteile. Ein erheblicher Vorteil der erfindungsgemäßen Zweiradkomponente wird durch die interne Luftführung erreicht, die zwei oder mehr Luftkanäle umfasst, wobei ein Luftkanal von der äußeren Oberfläche des Sondenkörpers bis zu einer inneren Kammer reicht und über wenigstens einen anderen Luftkanal bzw. Sensorkanal die innere Kammer mit dem Luftdrucksensor gekoppelt oder verbunden ist. Dadurch wird die äußere Oberfläche des Sondenkörpers nur über die Luftführung innerhalb des Sondenkörpers mit den Luftdrucksensoren verbunden. Der Sondenkörper wird insbesondere nicht temperiert und wird vorzugsweise nicht geheizt und wird auch nicht gekühlt. Das bedeutet, dass die Messsonde körperlich so beschaffen ist, dass in allen Wetterlagen eine effektive und wiederholbare Messung ermöglicht wird. Dazu wird das Eindringen von Wasser konstruktiv verhindert. Das wird vorteilhaft durch Luftkanäle mit geringeren Durchmessern erreicht.

Luftdrucksensoren sind in der Regel feuchtigkeitsempfindlich und sollten einem direkten Wasserkontakt besser nicht ausgesetzt werden. Durch die erfindungsgemäße Zweiradkomponente werden Luftdrucksensoren ausreichend geschützt. Die innere Kammer ermöglichte dabei die Aufnahme eventuell eindringenden Wassers, sodass eine Weiterleitung von eventuell in den Zufuhrkanal eindringenden Wassers oder auch anderen Substanzen bis zu den Luftdrucksensoren praktisch ausgeschlossen ist.

Insbesondere ist ein minimaler lichter Durchmesser wenigstens eines (oder wenigstens zweier) der Luftkanäle kleiner 2,0 mm, insbesondere kleiner 1,5 mm. Besonders bevorzugt beträgt ein minimaler lichter Durchmesser weniger als 1,25 mm. Besonders bevorzugt liegt ein minimaler lichter Durchmesser zwischen 0,25 mm und 1,5 mm und besonders bevorzugt zwischen 0,75 mm und 1,25 mm.

Unter einem minimalen lichten Durchmesser wird hier kein Durchmesser einer Engstelle verstanden, sondern ein lichter Durchmesser verstanden, der sich über eine Länge von mindestens 10% der Länge eines Luftkanals erstreckt. Wenn sich der lichte Durchmesser z. B. kontinuierlich von einem zum anderen Ende ändert, dann ist der minimale lichte Durchmesser der Durchmesser, der sich über den ersten Abschnitt von 10% der Länge durchschnittlich ergibt. Bei einem konstanten Querschnitt ist der minimale lichte Durchmesser gleich dem jeweils örtlichen Durchmesser.

Besonders bevorzugt ist eine Abmessung wenigstens eines (oder wenigstens zweier) der Luftkanäle an der äußeren Öffnung kleiner 1,5 mm und insbesondere kleiner 1,25 mm. Besonders bevorzugt beträgt ein minimaler lichter Durchmesser weniger als 1,25 mm. Besonders bevorzugt liegt eine Abmessung an der äußeren Öffnung zwischen 0,25 mm und 1,25 mm und besonders bevorzugt zwischen 0,5 mm und 1,0 mm. Geringe Durchmesser bzw. Abmessungen an der äußeren Öffnung der Luftkanäle bieten einen zuverlässigen Schutz vor dem Eintritt von Wasser in das Innere und sorgen deshalb für eine zuverlässige Funktion.

Vorzugsweise ist eine Abmessung an der äußeren Öffnung kleiner als ein durchschnittlicher oder typischer Durchmesser des entsprechenden Luftkanals. Der typische Durchmesser ist ein repräsentativer Durchmesser, der über den größten Anteil einer Länge eines Luftkanals vorliegt.

Durch die Abmessungen wird dafür gesorgt, dass das Eindringen von Wasser in die Messsonde jederzeit weitestgehend verhindert wird.

Vorzugsweise ist in einer inneren Kammer wenigstens ein Aufnahmeraum für (dennoch) eventuell eindringendes Wasser ausgebildet. Dadurch kann eventuell durch die äußere Öffnung an dem am Sondenkörper eingedrungenes Wasser zuverlässig gesammelt werden und der Luftdrucksensor wird ausreichend geschützt.

Vorzugsweise ist der Aufnahmeraum topfförmig ausgebildet. Dabei ist insbesondere sowohl der Eingang als auch der Ausgang des Aufnahmeraums von dem Boden des Aufnahmeraums beabstandet, um ein entsprechendes Reservoir für eventuell eindringendes Wasser zur Verfügung zu stellen.

In allen Ausgestaltungen ist es möglich und auch bevorzugt, dass die innere Kammer oder dass wenigstens eine innere Kammer wenigstens eine Zwischenwand aufweist. Durch eine Zwischenwand wird die innere Kammer in wenigstens zwei Teilkammern unterteilt. Die zwei Teilkammern sind vorzugsweise über wenigstens eine Verbindungsöffnung miteinander verbunden. Die Verbindungsöffnung der Teilkammern ist insbesondere an der Zwischenwand ausgebildet. Möglich ist es auch, dass die Verbindungsöffnung als Kanal ausgebildet ist. In der Regel ist es aber einfacher und deshalb bevorzugt, dass eine kleine Öffnung in der Zwischenwand als Verbindungsöffnung dient.

Wird eine innere Kammer durch eine Zwischenwand in zwei Teilkammern unterteilt, so werden verschiedene Aufnahmeräume zu Verfügung gestellt, was das Eindringen von Wasser weiter erschwert.

Insbesondere ist im bestimmungsgemäßen Gebrauch die Verbindungsöffnung vom unteren Ende der inneren Kammer beabstandet angeordnet. Dadurch wird im unteren Bereich ein Auffangraum für eingedrungenes Wasser gebildet. Es ist möglich und bevorzugt, dass insgesamt mehrere innere Kammern oder mehrere Teilkammern in dem Sondenkörper ausgebildet sind, die über Luftkanäle bzw. Zwischenkanäle miteinander verbunden sind. Insbesondere sind die Verbindungsöffnungen an mehreren hintereinander geschalteten Teilkammern versetzt zueinander angeordnet, wodurch das weitere Eindringen von Wasser erschwert wird.

Vorzugsweise ist an der Luftführung wenigstens eine Art von Labyrinthdichtung ausgebildet. Beispielsweise kann eine derartige Labyrinthdichtung durch mehrere hintereinandergeschaltete Kammern oder Teilkammern gebildet werden, wobei die Verbindungsöffnungen zwischen den einzelnen Teilkammern in seitlicher Richtung und/oder in der Höhe (im bestimmungsgemäßen Einbau) zueinander versetzt angeordnet sind. Vorzugsweise ist die Mehrzahl der Verbindungsöffnungen oder es sind alle Verbindungsöffnungen bzw. Mündungen der Zwischenkanäle von dem Boden der jeweiligen Kammer aus beabstandet angeordnet. Dadurch wird insbesondere eine im Zickzack ausgebildete Luftführung erreicht. Vorzugsweise ist in dem Bodenbereich der jeweiligen Kammer jeweils ein Aufnahmeraum für eventuell eingedrungenes Wasser ausgebildet. Dadurch wird das Eindringen von Wasser bis zu dem Sensorkanal, der schließlich zu dem Luftdrucksensor führt, weitestgehend vermieden.

In allen Ausgestaltungen ist es möglich, dass die Labyrinthdichtung zwei oder mehr innere Kammern und dazwischen angeordnete Luftkanäle und/oder zwei oder mehr Teilkammern und dazwischen angeordnete Verbindungsöffnungen oder Kanäle umfasst.

Die Zweiradkomponente ist vorzugsweise so ausgebildet, dass die Zuführung und alle Kammern einfach gereinigt (z. B. ausgeblasen o.ä.) werden können. Die Zweiradkomponente ist vorzugsweise einfach zu warten.

Vorzugsweise ist das Gehäuse und/oder der Sondenkörper aerodynamisch ausgebildet. Vorzugsweise ist der Sondenkörper lang gestreckt ausgebildet und weist eine im Wesentlichen rotationssymmetrische Außenfläche auf. An dem Sondenkörper ist vorzugsweise an der vorderen Spitze zentral eine Öffnung ausgebildet, die den Beginn des Zufuhrkanals definiert. Möglich ist es auch, dass in seitlichen Bereichen der Spitze des Sondenkörpers zwei oder mehr Öffnungen ausgebildet sind, die über entsprechende Luftkanäle mit einem oder mehreren Sensoren verbunden sind.

Es ist bevorzugt, dass der Sondenkörper wenigstens zu einem Teil und insbesondere zu einem erheblichen Teil oder überwiegend oder nahezu vollständig oder vollständig aus wenigstens einem Metall besteht.

Besonders bevorzugt ist wenigstens ein wesentlicher Teil des Sondenkörpers unter Ausbildung der Luftführung durch ein 3D-Druckverfahren insbesondere einstückig hergestellt. Durch ein solches dreidimensionales Druckverfahren wird eine effiziente Herstellung eines an sich komplexen Sondenkörpers ermöglicht. Dabei kann der Sondenkörper nahtlos ausgebildet werden und im Inneren eine Luftführung aufweisen, die mehrere Luftkanäle und mehrere Kammern und Teilkammern umfasst. Die Außenoberfläche kann mechanisch nachbearbeitet sein.

Vorzugsweise weist der Sondenkörper wenigstens zwei oder drei Öffnungen auf, denen wenigstens ein Luftdrucksensor oder auch zwei oder mehr Luftdrucksensoren oder jeweils ein Luftdrucksensor zugeordnet sind.

Der Sondenkörper ist vorzugsweise mit dem Gehäuse verbunden. Der Sondenkörper ist dabei vorzugsweise angesteckt. Insbesondere ist der Sondenkörper abnehmbar mit dem Gehäuse verbunden.

Besonders bevorzugt ist der wenigstens eine Luftdrucksensor in dem Gehäuse angeordnet. Zur Verbindung des Luftdrucksensors mit dem Sensorkanal kann innerhalb des Gehäuses ein entsprechender weiterer Sensorkanal vorgesehen sein.

Vorzugsweise ist in dem Gehäuse eine Recheneinrichtung angeordnet. In dem Gehäuse können weitere Sensoren angeordnet sein, um die Erfassung unterschiedlichster Daten zu ermöglichen.

Vorzugsweise sind die Abmessungen der äußeren Öffnungen an dem am Sondenkörper und der inneren Luftführung so ausgelegt, dass der resultierende Querschnitt der Luftkanäle (bzw. die Geometrie der Luftführung) klein genug ist, dass die Wasseroberflächenspannung an dem Querschnitt des Luftkanals bzw. der Luftkanäle der Kraft widersteht, die sich aus dem dynamischen Druck der Luft bei

Luftgeschwindigkeiten von bis zu 20 m/s und insbesondere wenigstens 30 m/s und vorzugsweise wenigstens 35 m/s oder bis zu 35 m/s ergibt. Dadurch wird das Eindringen von Wasser bis zu den Luftdrucksensoren verhindert. Ein erheblicher Vorteil dabei ist, dass durch die schon zuvor in dem Luftkanal vorhandene Luftmenge eine Art von Luftfeder entsteht, wenn Wasser in den entsprechenden Luftkanal eindringt. Da Wasser aufgrund der Wasseroberflächenspannung den vollständigen Kanalquerschnitt ausfüllt, kann sich das eingedrungene Wasser nur gegen die Federkraft der sich komponierenden Luft hinter dem Wasserpropfen weiter in den Kanal hinein bewegen. Deshalb steigt der Druck mit zunehmender Eindringtiefe an und es wird somit gleichzeitig eine bessere Absicherung erzielt.

In bevorzugten Weiterbildungen enthält jede Innenkammer einen Aufnahmeraum bzw. ein Volumen, in dem sich das Wasser, das durch die äußeren Öffnungen in die Messsonde eintritt, sammeln kann. Vorzugsweise ist im Wesentlichen jede Innenkammer oder jede Innenkammer so positioniert, dass der Aufnahmeraum vom Ausgang der inneren Kammer in Richtung des Luftdrucksensors entfernt ist.

Vorzugsweise ist im bestimmungsgemäßen Gebrauch der Zufuhrkanal ansteigend ausgebildet, sodass die äußere Öffnung des Zufuhrkanals tiefer angeordnet ist als der Mündungsbereich des Zufuhrkanals in eine innere Kammer. Vorzugsweise ist der Ausgang der letzten inneren Kammer in den Sensorkanal an einer hoch liegenden Stelle angeordnet, damit sich alle zuvor liegenden Aufnahmeräume zunächst mit eingedrungenem Wasser füllen müssen, bevor eingedrungenes Wasser durch den Sensorkanal weitergeleitet wird.

In allen Ausgestaltungen ist es bevorzugt, dass die Zweiradkomponente in einem vorderen Bereich des Fahrrads befestigt wird. Die Zweiradkomponente kann zum Beispiel mitlenkend am Lenker angebracht sein, sodass der Sondenkörper immer in die aktuelle Fahrtrichtung zeigt.

Vorzugsweise ist die Zweiradkomponente so klein und aerodynamisch wie möglich gestaltet, insbesondere so, dass ein minimaler Luftwiderstand entsteht, sowohl bei frontaler als auch bei seitlicher Anströmung.

Mit der Zweiradkomponente oder weiteren Teilen und Sensoren können verschiedene Daten erfasst werden, um die aktuelle Antriebsleistung, die aktuelle Fahrgeschwindigkeit, die aktuelle Steigung des Weges bzw. der Straße, die aktuelle Windgeschwindigkeit und Windrichtung zu bestimmen. Aus verschiedenen dieser Daten kann auf die aktuellen Fahrbedingungen zurückgeschlossen werden. Es ist möglich, den aktuellen Windwiderstand bei Fahren des Fahrrads inklusive einem gegebenenfalls darauf sitzenden Fahrer abzuleiten, um dem Fahrer die Bewertung seiner aktuellen Position auf dem Fahrrad oder auch die Verwendung verschiedener Komponenten zu erleichtern.

Die Erfindung wird insbesondere an wenigstens teilweise oder vollständig muskelbetriebenen Zweirädern und insbesondere Fahrrädern eingesetzt. Deshalb kann der Begriff Zweiradkomponente auch durchgängig durch den Begriff Fahrradkomponente ersetzt werden. Möglich und vorzugsweise vorgesehen ist der Einsatz insbesondere aber auch an sogenannten "Leichten Elektrofahrzeugen" (englisch: "Light Electric Vehicles" - abgekürzt LEV), was Elektrofahrzeuge mit zwei oder auch vier Rädern sind, die von einer Batterie, Brennstoffzelle oder einem Hybridantrieb angetrieben werden und im Allgemeinen weniger als 100 kg wiegen und vorzugsweise weniger als 80 kg und besonders bevorzugt weniger als 50 kg oder 30 kg wiegen. Besonders bevorzugt ist die Erfindung für den Einsatz an nach oben offenen Zweirädern oder Fahrrädern eingesetzt. Das sind Zweiräder ohne Dach.

Insbesondere umfasst die Zweiradkomponente eine Energiequelle wie eine Batterie. Besonders bevorzugt umfasst die Zweiradkomponente eine Anzeige und/oder eine Schnittstelle zu einer Anzeige. Es ist weiterhin möglich, dass wenigstens ein Feuchtesensor umfasst ist. Insbesondere kann auch wenigstens ein Temperatursensor umfasst sein. Mit einem Feuchtesensor und/oder einem Temperatursensor kann beispielsweise die Dichte der Luft ermittelt werden.

Ein Zweirad und insbesondere ein Fahrrad im Sinne der vorliegenden Erfindung weist vorzugsweise zwei Räder an zwei unterschiedlichen Achsen auf. Insbesondere sind die beiden Räder im bestimmungsgemäßen Gebrauch bei der Fahrt (wenigstens im Wesentlichen oder vollständig) hintereinander angeordnet.

Weitere Vorteile und Merkmale ergeben sich aus den Ausführungsbeispielen, die im Folgenden mit Bezug auf die beiliegenden Figuren erläutert werden.

In den Figuren zeigen:
- Figur 1: eine schematische Seitenansicht eines Rennrades auf einer ansteigenden Straße mit einer erfindungsgemäßen Zweiradkomponente;
- Figur 2: eine Zweiradkomponente in einer schematischen Seitenansicht;
- Figur 3: eine geschnittene Prinzipskizze einer Zweiradkomponente;
- Figur 4: eine weitere geschnittene Prinzipskizze einer Zweiradkomponente;
- Figur 5: ein schematisches Detail einer Zweiradkomponente;
- Figur 6: eine perspektivische Darstellung einer Messsonde einer Zweiradkomponente;
- Figur 7: die Messsonde nach Figur 6 in einer Seitenansicht;
- Figur 8: Vorderansicht von Figur 6;
- Figur 9: einen Schnitt durch Figur 6;
- Figur 10: einen Verlauf des Druckkoeffizienten auf einer Oberfläche eines angeströmten Körpers;
- Figur 11: einen Verlauf des mit einer Zweiradkomponente gemessenen absoluten Umgebungsdrucks über der zum Fahrrad relativen Luftgeschwindigkeit; und
- Figur 12 bis 14: einen Höhenverlauf einer Straße über der Strecke und beim Fahren darauf gemessene Werte.

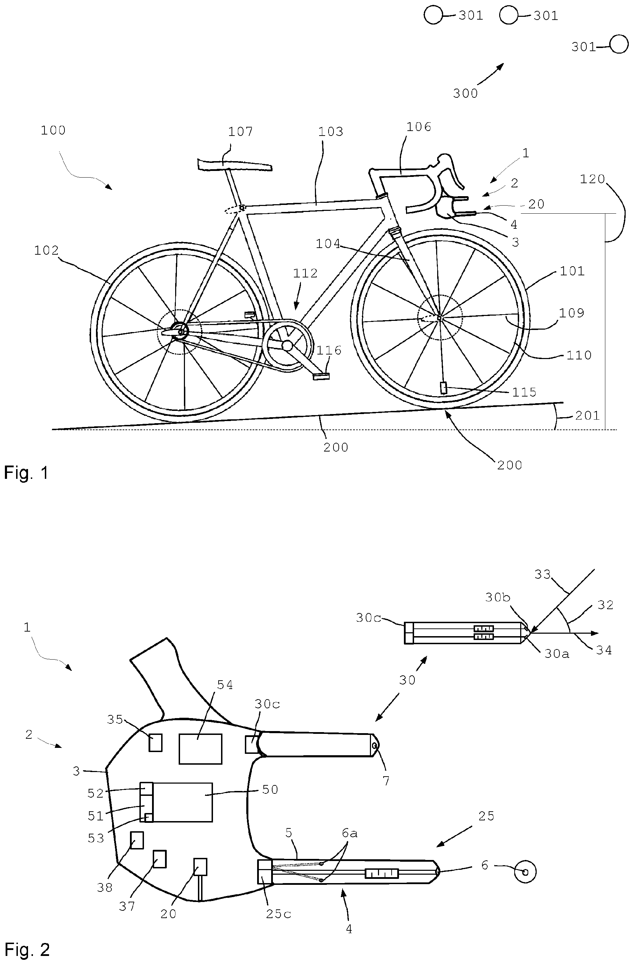

In Figur 1 ein Rennrad 100 dargestellt, die Erfindung kann auch bei einem Mountainbike eingesetzt werden. Das Rennrad 100 verfügt über ein Vorderrad 101 und ein Hinterrad 102. Die beiden Räder 101, 102 verfügen über Speichen 109 und eine Felge 110. Es können konventionelle Felgenbremsen oder auch andere Bremsen wie zum Beispiel Scheibenbremsen vorgesehen sein.

Ein Rad 100 verfügt über einen Rahmen 103, einen Lenker 106, einen Sattel 107 und eine Gabel. Zum Antrieb dient eine Tretkurbel 112 mit Pedalen. Gegebenenfalls kann ein elektrischer Hilfsantrieb an der Tretkurbel 112 und/oder den Rädern vorgesehen sein. Die Nabe der Räder kann z. B. jeweils über eine Steckachse oder einen Schnellspanner an dem Rahmen befestigt sein.

Das in Figur 1 dargestellte Rennrad 100 fährt hier auf einem Weg bzw. einer Straße 200 bergauf. Der Steigungswinkel 201 zeigt die vorliegende Steigung an. Über einen oder mehrere Geschwindigkeitssensoren 115 wird die Fahrgeschwindigkeit des Rennrades 100 auf dem Weg 200 ermittelt. Die Geschwindigkeit kann über Speichensensoren oder auch im Laufrad selbst und/oder über Satellitensysteme ermittelt werden. Über Leistungs- oder Kraftsensoren 116 an den Pedalen und/oder über entsprechende Sensoren an der Tretkurbel und/oder an der Hinterradnabe wird die Antriebsleistung des Rennrades 100 berechnet.

An dem Lenker 106 und/oder der Gabel ist die Zweiradkomponente 1 mit der Messeinrichtung 2 mitlenkend befestigt. Die erfassten Daten können in der Zweiradkomponente 1 bzw. der Messeinrichtung 2 der Zweiradkomponente 1 oder in einem separaten (Fahrrad-) Computer ausgewertet, gespeichert und verarbeitet werden.

Die Zweiradkomponente 1 umfasst eine Messeinrichtung 2 und ein Gehäuse 3, an dem eine oder mehrere Messsonden 4 angeordnet sind. Die Messsonde 4 kann als Pitot-Rohr ausgebildet sein und ein Maß für den Staudruck an dem vorderen Ende der Messsonde 4 bzw. dem Sondenkörper 5 erfassen. Durch eine innere Luftführung kann der Totaldruck einem Luftdrucksensor zugeleitet und dort erfasst werden. Durch Berücksichtigung des Umgebungsdrucks kann der Staudruck abgeleitet werden. Vorzugsweise wird ein Differenzdrucksensor eingesetzt, der eine Druckdifferenz zwischen dem Totaldruck am vorderen Ende und einem seitlichen lokalen statischen Druck (lokaler Umgebungsdruck) an der Messsonde 4 erfasst. Möglich ist es auch, dass zwei Absolutdrucksensoren eingesetzt werden und deren Differenz ermittelt wird, um den Staudruck zu berechnen.

Figur 2 zeigt eine vergrößerte Darstellung der Zweiradkomponente 1 aus Figur 1 und zeigt schematisch einige Komponenten bzw. Teile der Zweiradkomponente 1. Die Zweiradkomponente 1 weist eine Messeinrichtung 2 auf. In Fahrtrichtung gesehen ist am vorderen Ende der Zweiradkomponente 1 die Messsonde 4 mit dem Sondenkörper 5 angeordnet, um den Staudruck am vorderen Ende der Zweiradkomponente 1 und somit nahe dem vorderen Ende des Fahrrads 100 zu erfassen. Durch die Positionierung im vorderen Bereich wird eine mögliche Beeinflussung durch weitere Komponenten des Rennrades 100 im Wesentlichen vermieden.

Der Sondenkörper 5 weist am vorderen Ende die äußere Öffnung 6 auf. Die Öffnung 6 ist über eine später noch ausführlicher beschriebene Luftführung 10 im Inneren des Sondenkörpers 5 mit dem hier schematisch dargestellten Staudrucksensorsystem 25 verbunden. Eine Vorderansicht ist schematisch rechts neben der eigentlichen Zweiradkomponente abgebildet. Der runde Sondenkörper 5 mit der zentralen vorderen Öffnung 6 ist erkennbar.

Der Sondenkörper 5 der Messsonde 4 ist länglich geformt und über den wesentlichen Teil der Länge etwa zylindrisch ausgebildet. Beabstandet vom vorderen Ende und hier etwa in einem mittleren Abschnitt ist auf dem Umfang wenigstens ein Loch 6a ausgebildet. Hier ist das Loch 6a auf der Seitenwand des Sondenkörpers 5 mit dem Staudrucksensorsystem 25 verbunden. Ebenso ist die zentrale vordere Öffnung 6 mit dem Staudrucksensorsystem 25 verbunden.

Das Staudrucksensorsystem 25 umfasst hier einen Differenzdrucksensor 25c, der einen Differenzdruck zwischen den Öffnungen 6 und 6a erfasst. Dadurch wird ein dynamischer Differenzdruck erfasst, aus dem ein Staudruckwert bzw. Luftdruckwert abgeleitet wird. Über die Öffnungen 6a wird ein Wert für den lokalen statischen Druck erfasst, während über die Öffnung 6 der Totaldruck bei der Fahrt erfasst wird. Der Differenzdruck, der mit dem Differenzdrucksensor 25c des Staudrucksensorsystems 25 ermittelt wird, ist ein Maß für die relative Luftgeschwindigkeit, die frontal auf den Sondenkörper einströmt.

Hier sind vorzugsweise auf dem Umfang gleichmäßig mehrere Öffnungen 6a angeordnet, die innerhalb des Sondenkörpers 5 miteinander verbunden sind und somit einen statischen Durchschnittsdruck erfassen. In Figur 2 sind beispielhaft zwei Öffnungen 6a zu erkennen, die hier jeweils etwas oberhalb und etwa unterhalb der Mittellinie angeordnet sind. Die Öffnungen 6a können im Längsabschnitt der Öffnungen 6a miteinander verbunden sein oder über separate Kanäle mit dem Differenzdrucksensor 25c verbunden sein. Es können (symmetrisch verteilt) zwei oder mehr und insbesondere drei, vier, fünf, sechs, sieben oder auch acht oder mehr Öffnungen 6a auf dem Umfang verteilt angeordnet sein.

Im Inneren des Sondenkörpers 5 sind insbesondere an allen Kanälen die angedeuteten Kanäle ausgebildet, die das Eindringen von Wasser bis zu dem Sensor verhindern.

Die Zweiradkomponente 1 umfasst des Weiteren einen Luftdrucksensor 20 zur Erfassung des Umgebungsdrucks. Der Luftdrucksensor 20 zur Erfassung des Umgebungsdrucks kann an verschiedenen Stellen der Messeinrichtung 2 angeordnet sein. Jedenfalls sollte der Luftdrucksensor 20 nicht gleichfalls den Totaldruck erfassen, den der Staudrucksensor 25 an der vorderen Spitze der Messeinrichtung 2 erfasst.

Beispielsweise kann der Luftdrucksensor 20 zu Erfassung des Umgebungsdrucks (auch Absolutdrucksensor genannt) innerhalb des Gehäuses 3 und dort in einem unteren Bereich des Gehäuses 3 oder auch im hinteren Bereich des Gehäuses 3 angeordnet sein. Möglich ist es auch, dass der Luftdrucksensor 20 zur Erfassung des Umgebungsdrucks auf einer Seitenfläche oder an der Unterseite des Gehäuses 3 angeordnet ist oder eine Mündungsfläche aufweist. Jedenfalls wird mit dem Luftdrucksensor 20 ein Luftdrucksignal 21 für den Umgebungsdruck erfasst und nicht irgendein anderer Druck, der zwischen dem Totaldruck und dem Umgebungsdruck liegen könnte.

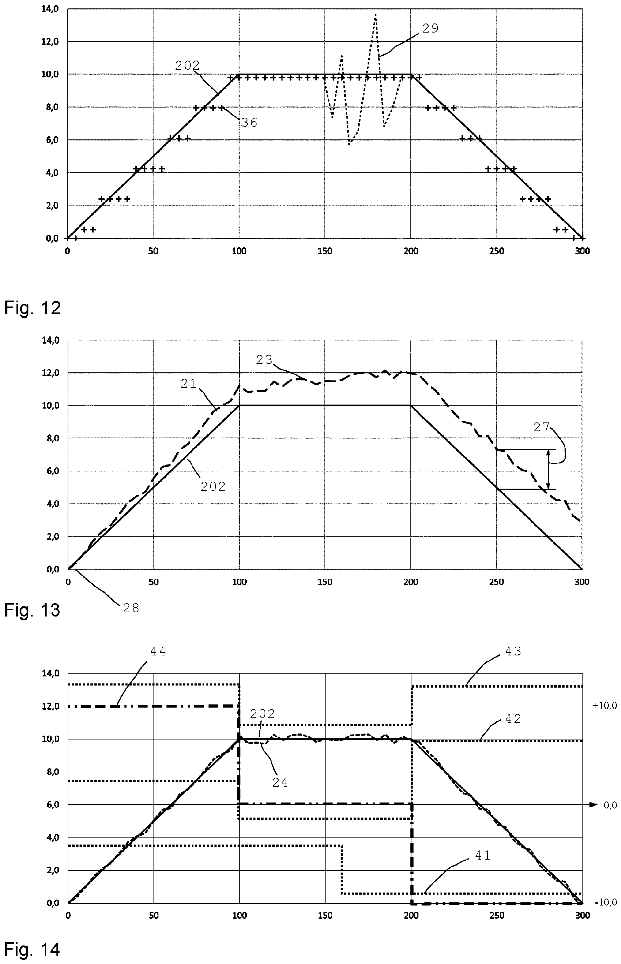

Die in Figur 2 dargestellt Zweiradkomponente 1 umfasst weiterhin ein Satellitensensor 35, mit dem von einem Satellitensystem 300 bzw. dessen Satelliten 301 (vergleiche Figur 1) Signale empfangbar sind, um daraus auf bekannte Art und Weise ein Höhensignal 36 (Figur 12) abzuleiten. Ein Feuchte- und/oder Temperatursensor 37 kann zu Bestimmung der Luftfeuchte und/oder Lufttemperatur vorhanden sein und auch zur Berechnung der Luftdichte der Umgebungsluft eingesetzt werden. Ein Beschleunigungssensor 38 dient zur Erfassung der Beschleunigungen des Rennrades 100.

Über eine Recheneinrichtung 50, die einen Speicher 51 und eine Datenschnittstelle und insbesondere eine Netzwerkschnittstelle 52 umfasst, können die aufgenommenen Daten verarbeitet, gespeichert und gegebenenfalls zu entfernten Gegenstellen übertragen werden. Die Datenschnittstelle kann auch eine Antenne zur Aufnahme und/oder Abgabe von Signalen umfassen. Darüber können Daten gegebenenfalls über Funk übertragen werden.

Als Energiequelle 54 kann eine Batterie oder ein Akku oder ein sonstiger Energiespeicher verwendet werden, der die benötigte Energie für die Sensoren, den Speicher und die Recheneinrichtung zur Verfügung stellt. Es kann auch eine Energieversorgung über das Zweirad erfolgen.

Ein Giersensor 30 weist hier einen Differenzdrucksensor 30c auf, der den Differenzdruck an den zwei Öffnungen 30a und 30b erfasst, die am vorderen Ende der Gierwinkelsonde angeordnet sind. Die Gierwinkelsonde ist am vorderen Ende mit zwei zueinander gewinkelt Flächen (insbesondere senkrecht zum Boden) ausgebildet, die hier unter einem Winkel von 90° zueinander ausgerichtet sind, und weist die zwei Öffnungen 30a und 30b auf. Aus den Messwerten wird ein Gierwinkel 32 abgeleitet.

Das Giersensorsystem weist einen ähnlichen Sondenkörper auf wie der des Staudrucksensorsystems 25. Im Inneren des Sondenkörpers des Giersensorsystems 30 sind zwei separate Luftführungen ausgebildet. An der vorderen Spitze sind gewinkelt zueinander zwei Öffnungen 30a, 30b vorgesehen, die insbesondere mit einem Differenzdrucksensor 30c oder separaten Drucksensoren verbunden sind, um einen Differenzdruck abzuleiten.

Um einen Überblick zu erleichtern, ist in der rechten Hälfte von Figur 2 eine Draufsicht auf den Sondenkörper des Giersensors 30 abgebildet, aus der erkennbar ist, das die Öffnungen 30a, 30b des Differenzdrucksensors 30c gewinkelt zueinander ausgerichtet sind.

Damit ist es möglich, aus der Fahrgeschwindigkeit 34 und den erfassten Werten die Windrichtung und die Windgeschwindigkeit 33 relativ zur Bewegung des Zweirades 100 zu ermitteln. Diese Windrichtung und Windgeschwindigkeit 33 entspricht dem Fahrtwind, dem der Fahrer unter dem Gierwinkel 32 ausgesetzt ist.

Möglich ist es auch, zwei oder gegebenenfalls auch mehrere Flächen zu haben, auf denen der Luftdruck gemessen wird, vorzusehen, die z. B. gewinkelt zueinander angeordnet sind, um aus den Differenzen der Messwerte einen Gierwinkel 32 abzuleiten.

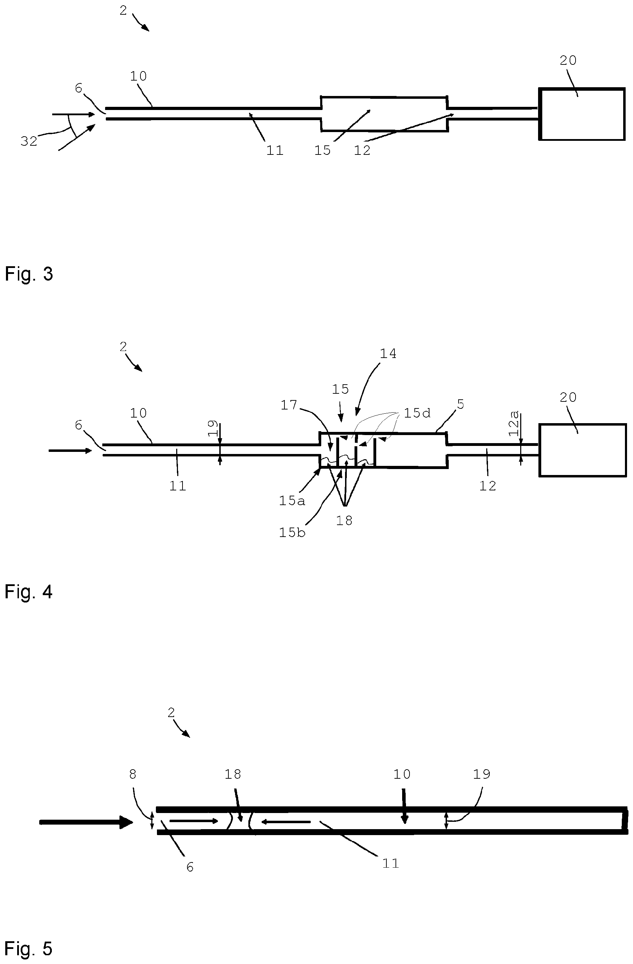

In den Figuren 3 bis 5 sind schematische Darstellungen einer Zweiradkomponente 1 bzw. einer Messeinrichtung 2 mit einer Messsonde 4 abgebildet. Figur 3 zeigt ein einfaches Beispiel einer Messsonde 4, wobei eine der Luftführungen 10 anschaulich dargestellt ist.

Der Übersichtlichkeit halber ist jeweils nur eine Luftführung 10 eingezeichnet, obwohl das Giersensorsystem 25 oder das Staudrucksensorsystem 30 zur Erfassung des Staudrucks vorzugsweise jeweils Differenzdrucksensoren und zwei oder mehr separate Luftführungen 10 umfassen. Verschiedene Luftführungen 10 sind innerhalb eines Sondenkörpers voneinander getrennt und weisen jeweils separate Kammern 15 und/oder Teilkammern und gegebenenfalls Zwischenwände 15c auf, um das Eindringen von Wasser und/oder Schmutz bis zu dem Drucksensor oder Differenzdrucksensor zu verhindern.

Am vorderen Ende des Sondenkörpers 5 ist die äußere Öffnung 6 ausgebildet, an die sich die Luftführung 10 und zunächst der Luftkanal 11 als Zufuhrkanal anschließt. Der Luftkanal 11 führt bis zu der Kammer 15, die einen Aufnahmeraum für eventuell eingedrungenes Wasser bereitstellt. Ein typischer Durchmesser 19 des Luftkanals 11 beträgt in einer bevorzugten Ausgestaltung etwa 1 mm (+/-20%). Schon durch den geringen Durchmesser bedingt, kann nur sehr wenig Wasser eindringen.

Am rückwärtigen Ende der Kammer 15 schließt sich der Luftkanal 12 an, der als Sensorkanal ausgebildet ist und bis zum Luftdrucksensor 20 führt. Auch der typische Durchmesser 12a des Sensorkanals 12 beträgt hier in einer bevorzugten Ausgestaltung etwa 1 mm (+/-20%). Durch den Aufbau der Luftführung 10 und den geringen Durchmesser der Luft- und Sensorkanäle bedingt. wird ein zuverlässiger Schutz des Luftdrucksensors 20 vor eindringendem Wasser gewährleistet.

Zum Schutz vor dem Eintritt von Wasser trägt auch bei, dass die äußere Öffnung 6 des Luftkanals 11 eine Abmessung bzw. einen (im Vergleich zum Durchmesser der Luftkanals 11 noch etwas kleineren) Durchmesser 8 aufweist. Der Durchmesser 8 ist hier etwa 20% kleiner als der typische Durchmesser 19 des Luftkanals 11. Durch eine nochmals kleinere äußere Öffnung 8 wird ein noch besserer Schutz vor dem Eindringen von Wasser erreicht.

Dadurch kann auf thermische Maßnahmen wie eine Beheizung des Sondenkörpers 5 verzichtet werden. Das Innere bleibt im Betrieb weitestgehend von Wasser frei. Auf das äußere Reinigen des Fahrrads mit einem Hochdruckreiniger sollte aber wenigstens im Bereich des Sondenkörpers 5 dennoch verzichtet werden.

Zunächst müsste der in der Kammer 15 ausgebildete Aufnahmeraum mit Wasser gefüllt werden, bevor danach Wasser in den Sensorkanal 12 eindringt. Aufgrund der geringen Abmessungen und der Oberflächenspannung von Wasser bildet eindringendes Wasser dort einen Pfropfen, der den Kanal dicht abschließt und damit die sich dahinter befindliche Luftmenge in dem Sensorkanal 12 einschließt. Damit das Wasser weiter in den Sensorkanal 12 eindringt, muss deshalb das eingeschlossene Luftvolumen komprimiert werden, was dem eingedrungenen Wasser eine entsprechende Gegenkraft entgegensetzt. Dadurch wird das Eindringen von Wasser bis zu dem Luftdrucksensor 20 weitestgehend vermieden.

In entsprechender Weise wie in den Figuren 3 bis 9 dargestellt, können die Sensoren 25, 30 angepasst ausgebildet sein.

Figur 4 zeigt eine Variante, bei der die innere Kammer 15 in mehreren Teilkammern 15a, 15b etc. aufgeteilt ist. Dazu sind hier Zwischenwände 15c vorhanden, die die Kammer 15 in die Teilkammern aufteilt.

In den Teilkammern 15a, 15b sind jeweils Aufnahmeräume 17 ausgebildet, in denen sich jeweils eingedrungenes Wasser 18 sammeln kann.

In den Zwischenwänden 14 der Kammer 15 sind Verbindungsöffnungen 15d ausgebildet, die die Teilkammern 15a, 15b etc. nacheinander und (wie Perlen auf einer Schnur) miteinander verbinden. Die Verbindungsöffnungen sind vom Boden der jeweiligen Kammern bzw. Teilkammern beabstandet angeordnet, um entsprechende Aufnahmeräume zu Verfügung zu stellen. In den Zwischenwänden sind Verbindungsöffnungen so angeordnet, dass diese nicht miteinander fluchten, sondern seitlich und/oder in der Höhe zueinander versetzt angeordnet sind. Dabei ist vorzugsweise jede einzelne Verbindungsöffnung aber vom Boden des jeweiligen Aufnahmeraums beabstandet angeordnet.

Insgesamt entstehen dadurch zwei oder mehr miteinander verbundene Kammern oder Teilkammern und mit der entsprechenden Luftführung dazwischen eine Art von Labyrinthdichtung 14, die besonders zuverlässig den Luftdrucksensor 20 vor eindringendem Wasser schützt. Die Luftführung kann im Zuge von Wartungsarbeiten oder nach jeder Fahrt vollständig oder teilweise gereinigt werden. Beispielsweise kann die Messsonde 4 demontiert und durchgespült und getrocknet und/oder mit (insbesondere ölfreier) Druckluft ausgeblasen werden.

Figur 5 zeigt einen Abschnitt eines Luftkanals 11, 12 oder 13, wobei ein Wassertropfen 18 in den Luftkanal beispielhaft eingezeichnet ist. Im Inneren weisen die Luftkanäle einen Durchmesser bzw. Querschnitt 19 auf. Der Durchmesser 19 beträgt insbesondere zwischen 0,5 mm und 2 mm. Hier im konkreten Beispiel liegt der lichte Durchmesser 19 bei 1 mm. Die äußere Öffnung 6 weist eine Abmessung 8 auf, die vorzugsweise geringer ist als der lichte Durchmesser 19. Hier liegt der Durchmesser 8 der äußeren Öffnung bei vorzugsweise 0,8 mm.

Die Abmessungen 8 und 19 sind so aufeinander und die Eigenschaften von Wasser abgestimmt, dass eingedrungenes Wasser im Inneren eines Luftkanals einen Wasserpfropfen 18 bildet, wie es in Figur 5 eingezeichnet ist. Der Pfropfen kann nur soweit in den Kanal eindringen, bis ein Gleichgewicht der durch das komprimierte Luftvolumen erzeugten Kraft und der durch den Totaldruck hervorgerufenen Kraft vorliegt. Die geringeren Durchmesser tragen erheblich zur Abdichtung bei.

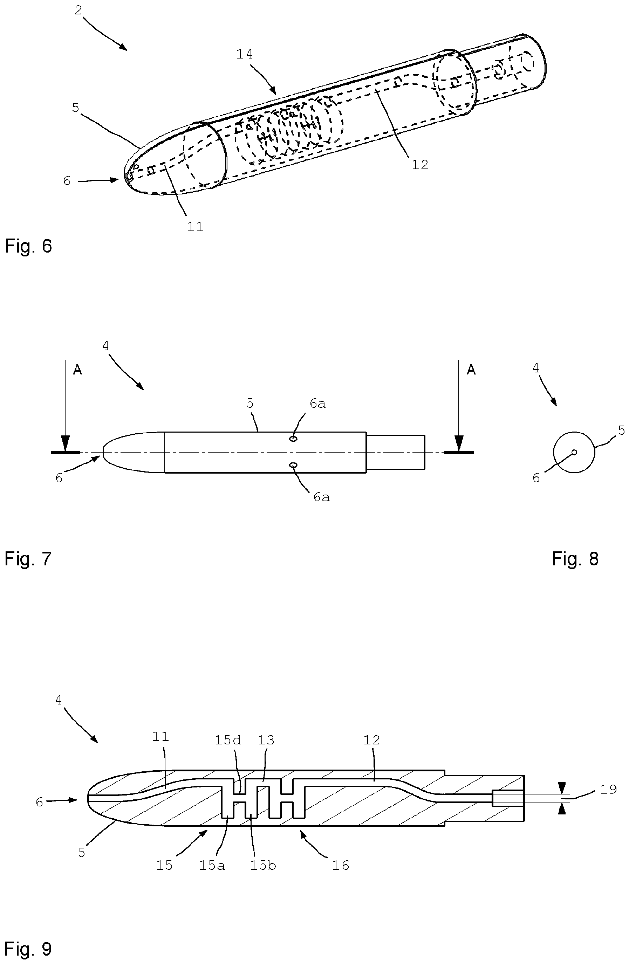

In den Figuren 6 bis 9 ist ein konkreteres Ausführungsbeispiel der Messsonde 4 mit einem Sondenkörper 5 dargestellt. Figur 6 zeigt dabei eine perspektivische Darstellung, wobei eine der Luftführungen 10 im Inneren des Sondenkörpers 5 gestrichelt eingezeichnet ist, um einen schematischen Überblick zu geben. Am vorderen Ende ist die äußere Öffnung 6 an der Spitze des Sondenkörpers 5 ausgebildet. Es schließt sich der Zufuhrkanal 11 als Luftkanal an. In einem zentralen Bereich ist eine Labyrinthdichtung 14 umfasst, die sich im hinteren Bereich des Sensorkanals 12 als Luftkanal anschließt.

Figur 7 zeigt eine Seitenansicht und Figur 8 den Schnitt A - A aus Figur 7. In Figur 7 sind zwei der hier z. B. insgesamt vier Öffnungen 6a (möglich sind auch drei oder fünf oder sechs oder mehr Öffnungen) auf dem seitlichen Umfang des Sondenkörpers 5 eingezeichnet, über die der statische Druck aufgenommen wird. Am hinteren Ende des des Sondenkörpers 5 wird dann vorzugsweise ein Differenzdrucksensor angeordnet, der einen Differenzdruck des Totaldrucks und des über dem Umfang des Sondenkörpers 5 gemittelten lokalen statischen Drucks (lokaler über den Umfang des Sondenkörpers gemittelter Umgebungsdruck) erfasst. Möglich ist es auch, zwei separate Luftdrucksensoren einzusetzen, die zur Bestimmung des Staudrucks eingesetzt werden.

In Figur 8 ist an der vorderen Spitze die Öffnung 6 zu erkennen.

Figur 9 zeigt einen Querschnitt durch den Sondenkörper 5, wobei hier erkennbar ist, dass im Inneren des Sondenkörpers 5 die Luftführung 10 verläuft und hier zwei Kammern 15,16 umfasst, die jeweils Teilkammern 15a und 15b aufweisen, insgesamt also hier vier (Teil-) Kammern. Im Querschnitt ist jede der Kammern 15, 16 etwa "H"-artig geformt, wobei die Zufuhr durch den Zufuhrkanal 11 am oberen Ende des "H" erfolgt. Auch die Verbindungen zu der zweiten Kammer 16 und dem Sensorkanal 12 starten jeweils am oberen Ende der Kammern 15, 16. Die Teilkammern 15a, 15b sind in einem mittleren bis oberen Bereich über eine Verbindungsöffnung 15d miteinander verbunden. Hier kann eine solche Verbindungsöffnung 15d auch als Zwischenkanal bezeichnet werden. Durch die Konstruktion können die unteren Schenkel der "H"-förmigen Kammern 15, 16 jeweils als Aufnahmeräume für eventuell eingedrungenes Wasser 18 eingesetzt werden.

Der Giersensor 30 ist entsprechend aufgebaut und umfasst einen Sondenkörper mit zwei Öffnungen und zwei Luftführungen und vorzugsweise einem Differenzdrucksensor 30c oder zwei Drucksensoren, um einen Wert für den Differenzdruck zu ermitteln..

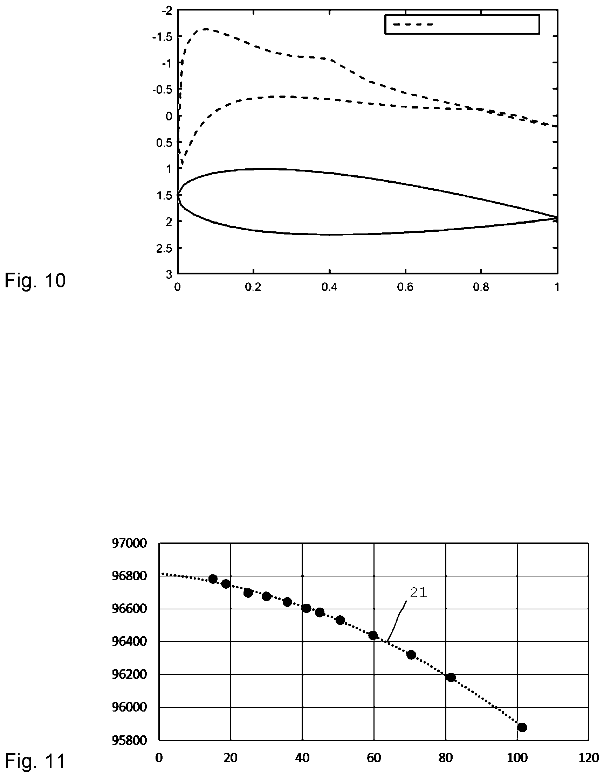

Figur 10 zeigt ein Diagramm mit der Druckverteilung um die Oberfläche eines Objektes herum, wenn auf das Objekt von vorne Luft einströmt. Figur 10 zeigt zwar einen Schnitt durch eine Tragfläche, aber grundsätzlich hängt auch bei anderen Objekten der Druck auf eine Oberfläche von dem Anströmwinkel und den Eigenschaften des Objektes ab. Während das Objekt hier mit einer durchgezogenen Linie eingezeichnet ist, ist der Druckkoeffizient gestrichelt eingezeichnet, der repräsentativ für den auf die Oberfläche des Objektes lokal einwirkenden Druck ist.

Aus Figur 10 ergibt sich in an sich bekannterweise, dass der lokale Druck auf die Oberfläche eines Objektes von der Position auf der Oberfläche des Objektes abhängt. Damit kann der lokale Druck größer oder kleiner als der normale Umgebungsdruck in Ruhe sein (und hängt auch noch von der Luftgeschwindigkeit ab).

Die Positionsabhängigkeit ist ein Problem, wenn mit einem sich relativ zu der Umgebungsluft bewegenden Fahrzeug - wie einem Fahrrad - ein Umgebungsdruck erfasst werden soll. Selbst im Inneren eines Objektes herrscht nicht der normale Umgebungsdruck vor, sondern wird durch die Fahrgeschwindigkeit, die Windgeschwindigkeit, die Windrichtung und auch durch die Konstruktion des Objektes beeinflusst.

Soll an einem Fahrrad einen Luftwiderstandsbeiwert ermittelt werden, müssen die nötigen Sensorwerte möglichst exakt erfasst werden. Dazu ist es von großem Vorteil, wenn die Steigung eines Weges und/oder auch die Windrichtung möglichst genau erfasst werden.

Figur 11 zeigt beispielhaft den mit einer Zweiradkomponente 1 direkt mit dem Luftdrucksensor 20 gemessenen "Umgebungsdruck" über der Luftgeschwindigkeit bzw. der Relativgeschwindigkeit des Fahrrads zum Wind. Dabei wird der Druck in Newton pro Quadratmeter (N/m2 bzw. Pa) über der Geschwindigkeit in Stundenkilometer aufgetragen. Es zeigt sich in diesem konkreten Fall, dass der Verlauf der bei tatsächlich gleichem Umgebungsdruck gemessenen Luftdrucksignale 21 stark von der Relativgeschwindigkeit abhängt. Mit zunehmender Luftgeschwindigkeit wird das gemessene Luftdrucksignal 21 kleiner. Der Unterschied im dargestellten Geschwindigkeitsbereich von 0 bis 100 km/h beträgt hier etwa 10 mbar bzw. 1000 Pa.

Der konkrete Verlauf hängt von der Anordnung des Luftdrucksensors zur Messung des Umgebungsdrucks, von der genauen Ausgestaltung der Messeinrichtung bzw. der Zweiradkomponente 1 und auch von der Windrichtung ab. Der Effekt kann aber unabhängig von einer gewählten Position nicht grundsätzlich vermieden werden. Auch wenn, wie hier, der Luftdrucksensor 20 für den Umgebungsdruck innerhalb des Gehäuses 3 angeordnet wird, kann es aufgrund von relativem Fahrtwind und relativer Richtung der Luft zu Beeinträchtigungen der Messqualität kommen. Es können dynamische Effekte auftreten, die den gemessenen Wert erhöhen oder verringern. Die Luft kann sich vor dem Eingang zum Sensor aufstauen oder durch einen Bernoulli-Effekt kann der gemessene Druckwert 21 kleiner sein, als der echte Umgebungsdruck.

Die Zweiradkomponente 1 umfasst im Speicher 51 der Recheneinrichtung Kalibrierdaten 53, mit denen das zunächst mit einem Luftdrucksensor 20 erfasste Luftdrucksignal 21 aufgrund von Messdaten oder Erfahrungsdaten korrigiert werden kann. Die Verwendung der mit dem Staudrucksensorsystem 25 erfassten Staudruckwerte 26 ist sehr vorteilhaft. Das Ergebnis kann unter Berücksichtigung des mit dem Gier-Sensor 30 erfassten Gierwinkels 32 nochmals verbessert werden.

Damit wird eine erheblich verbesserte Bestimmung der aktuellen Höhe des Rennrades 100 ermöglicht und es kann die Steigung bzw. der Steigungswinkel 201 eines Weges 200 mit erheblich verbesserter Genauigkeit abgeleitet werden.

Da der Neigungswinkel bzw. die Steigung oder das Gefälle eines Weges einen erheblichen Einfluss auf die benötigte Antriebsleistung hat, kann somit auch ein Luftwiderstandsbeiwert mit erheblich verbesserter Genauigkeit vermittelt werden. Eine negative Steigung wird meist Gefälle genannt. Auch bei einem Gefälle hat der Luftwiderstandsbeiwert einen großen Einfluss.

Weiterhin hat auch der Rollwiderstand Einfluss auf die benötigte Leistung. Dazu können weitere Messwerte aufgenommen und analysiert werden oder es wird auf zuvor erfasste Werte zurückgegriffen. Der Rollwiderstand wird durch die verwendeten Reifen, den Reifenluftdruck, das Gewicht des Fahrrads und des Fahrers und die Fahrbahnbeschaffenheit beeinflusst und kann ermittelt, berechnet und/oder abgeschätzt werden.

Zur Aufnahme entsprechender Kalibrierdaten 53 können entweder im Windkanal oder auf geeigneten Straßen bei geeigneten Umgebungs- und Windbedingungen entsprechende Daten erfasst und ausgewertet werden. Die Kalibrierdaten 53 können anschließend im normalen Betrieb verwendet werden, um die Genauigkeit der Messergebnisse und der abgeleiteten Werte zu vergrößern. Die Kalibrierdaten 53 für die Kalibriermatrix werden entweder aus Tests im Windkanal oder aus Fahrversuchen ohne Höhenänderung in einem Bereich von Luftgeschwindigkeiten und Gierwinkeln abgeleitet.

Die Figuren 12 bis 14 zeigen den Höhenverlauf eines Weges 200 über der Strecke und beim Fahren darauf gemessene und abgeleitete Werte.

In Figur 12 zeigt die durchgezogene Linie den tatsächlichen Höhenverlauf 202 des Weges 200 in Metern (relativ zum Ausgangspunkt) über der dargestellten Länge in Metern dar. Mit Kreuzen markiert gekennzeichnet sind einzelne Messpunkte, die während der Fahrt über die dargestellte Strecke ermittelt und aufgezeichnet wurden. Die eingezeichneten Messwerte sind Höhensignale 36, die durch einen Satellitensensor 35 ermittelt wurden.

Beispielsweise kann dazu ein GPS-Sensor oder ein sonstiger Satellitensensor 35 eines globalen Navigationssatellitensystems (englisch: "global navigation satellite system" oder GNSS) eingesetzt werden. Möglich sind auch Systeme mit Pseudosatelliten, die ein lokales Satellitensystem zur Verfügung stellen und eine entsprechende Triangulation der Höhe und/oder Position erlauben.

Deutlich erkennbar ist die hohe Genauigkeit des Satellitensensors 35 und der Höhensignale 36. Es ist aber auch direkt erkennbar, dass die Auflösung der Höhensignale 36 des Satellitensensors 35 relativ grob ist. Hier wird jeweils ein Höhenunterschied von knapp 2 m erkannt. Eine Auflösung von 2 m reicht für die Berechnung einer Steigung zur Bestimmung eines Luftwiderstandsbeiwertes beim Betrieb von Fahrrädern nicht aus.

Deshalb werden bei alleiniger Verwendung von Satellitensensoren 35 zur Ermittlung einer lokalen Steigung keine befriedigenden Ergebnisse erzielt, wenn daraus beispielsweise ein Luftwiderstandsbeiwert errechnet werden soll. Auch eine Interpolation zwischen den einzelnen Messwerten bringt nicht die benötigte Genauigkeit, da sich der Höhenverlauf vieler Wege erheblich von der hier dargestellten besonders einfachen Teststrecke unterscheidet. So kann sich die Steigung lokal schon auf einem Meter oder auf wenigen Metern erheblich ändern.

In Figur 12 ist zusätzlich noch der Verlauf des gemessenen Umgebungsdrucks bei einem Störereignis gestrichelt eingezeichnet. Beispielsweise können solche Störsignale 29 bei einem vorbeifahrenden Fahrzeug und insbesondere einem vorbeifahrenden Lastkraftwagen auftreten. Dabei weicht der gemessene Umgebungsdruck und die daraus berechnete Höhe erheblich von der tatsächlichen Höhe ab. Solche Ereignisse können über eine interne Filterung herausgerechnet werden. Dabei werden vorausgehende und folgende Werte erfasst und berücksichtigt und mit der Höhenbestimmung über GPS verrechnet. Der typische Verlauf mit sich abwechselnden Druckspitzen und Druckminima erleichtert die Filterung. Über die Filterung können so auch Fehlberechnungen der Windrichtung und der Windgeschwindigkeit vermieden werden.

Figur 13 zeigt die gleiche Strecke wie Figur 12, wobei einerseits wiederum der tatsächliche Höhenverlauf 202 der Teststrecke und andererseits ein über ein Luftdrucksignal 21 eines Luftdrucksensors 20 abgeleitetes Höhenprofil eingezeichnet ist.

Am Anfang wird ein Referenzwert 28 aufgenommen, der dann zur Bestimmung einer Höhendifferenz verwendet wird. Erkennbar ist, dass zu Beginn der Messung der über den Luftdrucksensoren gemessene Verlauf mit dem tatsächlichen Höhenverlauf 202 sehr gut übereinstimmt. Bei etwa der Mitte beträgt der Unterschied der Höhe beim Wert 23 schon fast 2 m.

Mit zunehmender Strecke ergibt sich aber ein systematischer Versatz bzw. ein Auseinanderlaufen des mit dem Luftdrucksensor 20 gemessenen Höhenverlaufs mit dem tatsächlichen Höhenverlauf 202. Das liegt daran, dass der Luftdrucksensor 20 nicht die Höhe direkt, sondern ein Maß für den Umgebungsdruck erfasst. Der absolute Umgebungsdruck hängt zwar auch von der Höhe ab, kann sich aber auch z. B. wetterbedingt ändern. Fällt nun der Luftdruck beim Fahren auf der Strecke oder steigt der Luftdruck währenddessen an, so können die damit bestimmten Werte auseinanderdriften. Das zeigt hier beispielhaft der Wert 27. Auch hier ist wieder das Ergebnis, dass die Werte nicht so genau sind, um einen hochqualitativen Luftwiderstandsbeiwert zu ermitteln. Dazu ist eine höhere Genauigkeit der Höhenerfassung sinnvoll.

Figur 14 zeigt schließlich einerseits wiederum den tatsächlichen Höhenverlauf 202 der Teststrecke und andererseits einen über die verschiedenen Sensoren bzw. mit den Messergebnissen der verschiedenen Sensoren korrigierten Verlauf der Höhenwerte 24.

Dazu werden die Luftdrucksignale 21 des im Inneren des Gehäuses 3 der Messeinrichtung 2 angeordneten Luftdrucksensor 20 mittels der mit dem Staudrucksensorsystem 25 erfassten Staudruckwerte 26 und der mit den Giersensor 30 erfassten Gierwinkelwerte 31 und gemäß der Kalibrierdaten 52 nach dem grundsätzlichen Prinzip der Darstellung von Figur 11 korrigiert, um ein weitestgehend korrektes Maß für den jeweiligen Höhenwert 24 zu erhalten.

Außerdem wird zusätzlich zur Erfassung von den Luftdrucksignalen 21 zur Bestimmung von Höhenmaßen auch der Satellitensensor 35 eingesetzt. In periodischen Intervallen wird der hochgenaue Satellitensensor 35 eingesetzt, um einen Vergleichswert zu erhalten. Weicht der über die verschiedenen Luftdrucksensoren 20, 25 und 30 ermittelte Höhenwert 24 von dem Höhensignal 36 des Satellitensensors 35 signifikant ab, wird ein neues Referenzsignal 28 abgeleitet, wodurch sich mit dem entsprechenden Luftdrucksignal 21 ein zutreffend korrigierter Höhenwert 24 ergibt. Um eine Rekalibrierung aufgrund von verrauschten Messwerten zu vermeiden, wird nur korrigiert, wenn sich Differenzen über einen signifikanten Zeitraum ergeben.

Über dieses Verfahren werden die Vorteile der hohen Genauigkeit von Satellitensensoren 35 mit den Vorteilen der hohen Auflösung von Luftdrucksensoren 21 kombiniert. Gleichzeitig werden die Nachteile der niedrigen Auflösung von Satellitensensoren 35 und von möglichen Luftdruckschwankungen bei Luftdrucksensoren 20 vermieden. Es ergibt sich, wie in Figur 14 zu erkennen, eine sehr hohe Übereinstimmung des effektiven Verlaufs der Höhenwerte 24 mit dem tatsächlichen Höhenverlauf 202 der Teststrecke.

Ein (erstes) Referenzsignal 28 kann beispielsweise zu Beginn einer Fahrt eingegeben oder erfasst werden oder dann, wenn die Höhe bekannt ist. Durch Differenzbildung von dem Luftdrucksignal 21 bei der Fahrt und dem Referenzsignal 28 kann ein Maß für die aktuelle Höhe ermittelt werden. Das Referenzsignal 28 kann zunächst ermittelt werde, indem ein anfängliches Luftdrucksignal 21 ermittelt wird und für folgende Messungen als Referenzsignal 28 verwendet wird. Das zugehörige Referenzsignal 28 kann auch eingegeben oder per Satellitensensor 35 erfasst werden. Im Laufe der Fahrt kann das Referenzsignal 28 periodisch und in unregelmäßigen Zeitabständen aktualisiert werden.

Die Korrektur des zur Berechnung eines Höhenmaßes 23 verwendeten Referenzsignals 28 kann beispielsweise durchgeführt werden, wenn die Summe der Abweichungen zwischen den Höhensignalen 36 des Satellitensensors 35 und den ermittelten Höhenwerten 24 über einen gewissen Zeitraum ein vorbestimmtes Maß oder eine vorbestimmte Schwelle überschreiten. Beispielsweise kann über eine bestimmte Strecke oder nach einem bestimmten Zeitraum ein Mittelwert gebildet werden, der entsprechend zum Vergleich herangezogen wird.

Vorzugsweise werden Höhensignale 36 zwischen etwa 20 oder 30-mal pro Sekunde und etwa 3 bis 5 mal pro Minute gemessen, insbesondere mit einer Frequenz von etwa 0,1 Hz. Die Frequenz, mit der ein aktuelles Höhenmaß aus einem aktuellen charakteristischen Luftdrucksignal für einen Umgebungsdruck erfasst wird, ist vorzugsweise höher und liegt insbesondere zwischen 0,1 Hz und 1 kHz und vorzugsweise zwischen 1 Hz und 100 Hz, besonders bevorzugt bei etwa 50 Hz.

Besonders bevorzugt ist ein Verhältnis der Messfrequenz des Luftdrucksignals 21 für einen Umgebungsdruck zu der Messfrequenz eines Höhensignals 36 größer 10 und insbesondere größer 100 und vorzugsweise kleiner 5000. Dadurch kann ein hohes Maß an Genauigkeit erreicht werden, während der Energiebedarf gering bleibt.

Zusätzlich sind in Figur 14 drei Verläufe 41, 42 und 43 der Messfrequenzen dargestellt. Die Messfrequenz für die Erfassung der Signale und insbesondere die Erfassung der Höhensignale oder die Erfassung der Luftdrucksignale hängt von den aktuellen Fahrbedingungen ab und kann mit der Steuereinrichtung 40 eingestellt und bei Bedarf verändert werden. So wird die Messfrequenz insbesondere an Steigungen und besonders bevorzugt an Gefällen größer eingestellt als auf geraden Strecken. Die Kurven 41 bis 43 zeigen jeweils die Messfrequenz über der Strecke und sind hier der besseren Übersichtlichkeit halber höhenversetzt eingezeichnet, um die Verläufe jeweils getrennt darzustellen.

Der erste Messverlauf 41 zeigt ein Beispiel mit grundsätzlich konstanter Messfrequenz, bei dem der Zustand der Energieversorgung etwa auf der Mitte der Strecke eine Schwelle unterschreitet. Danach werden Energiesparmaßnahmen eingeleitet und die Messfrequenz wird deutlich reduziert. Es ist möglich, dass auf dem reduzierten Niveau auch noch eine Variierung der Messfrequenz in Abhängigkeit von aktuellen Fahrbedingungen erfolgt, beispielsweise eine Erhöhung bei zunehmender Geschwindigkeit oder bei Steigungen oder bei Gefälle. In der Ebene kann die Messfrequenz auch noch weiter reduziert werden.

Der zweite Messverlauf 42 zeigt eine Steuerungsvariante, bei der im Bereich der ersten Steigung eine erhöhte Messfrequenz eingestellt wird. Nach Erreichen des mittleren Plateaus wird in dem dort ebenen Bereich die Messfrequenz erheblich reduziert (z. B. Faktor 1/2). Bei Beginn des Gefälles wird die Messfrequenz stark erhöht, damit bei der höheren Fahrgeschwindigkeit bergab eine sehr hohe Genauigkeit erzielt wird.

Der dritte Messverlauf 43 zeigt ein Beispiel, bei dem im Bereich der Neigungen des Weges (Steigung/Gefälle) mit erhöhter Messfrequenz gemessen wird, während in der Ebene mit reduzierter Messfrequenz gemessen wird. Dadurch wird im Bereich der Neigungen eine erhöhte Genauigkeit erreicht und es wird im Bereich der Ebene weniger Energie benötigt. Die Verläufe 41 bis 43 können insbesondere nicht nur die Messfrequenz über dem Weg, sondern können auch Verläufe der Messfrequenz über der Fahrzeit wiedergeben.

Die schematisch eingezeichneten Verläufe 41 bis 43 zeigen die Messfrequenz über dem Weg für eine konstant gefahrene Geschwindigkeit wird. Bei unterschiedlichen Fahrgeschwindigkeiten krümmen sich die Kurven entsprechend.

Es ist bevorzugt, dass die Messverläufe 41 bis 43 zu Beginn beim Zeitpunkt 0 jeweils die gleiche Messfrequenz aufweisen. Die absolute Höhe ist hier entsprechend versetzt dargestellt, um die Verläufe besser grafisch unterscheiden zu können.

Weiterhin ist in Figur 14 der Steigungsverlauf 44 über der Messstrecke strichpunktiert eingezeichnet. Zu Beginn ist der Verlauf der Steigung über das erste Drittel der Messstrecke konstant hoch. Dort liegt die Steigung bei einem Wert (rechte Skala) von +10,0. Im zweiten Drittel in der Ebene ist die Steigung 0,0 und im dritten Drittel liegt ein Gefälle mit einer Steigung von hier -10,0 vor. Über die periodisch erfassten Luftdrucksignale 21 und die zugehörigen Streckendaten kann ein Steigungswert 201 abgeleitet werden. Dazu werden die Daten zuvor noch gemittelt und gefiltert.

Mit dem bekannten Gewicht des Fahrrads und des Fahrers können dann Leistungsdaten aus dem aktuellen Steigungswert und dem aktuellen Geschwindigkeitswert abgeleitet werden. Dazu wird berücksichtigt, ob und wie das Fahrrad beschleunigt wird.

Unter Berücksichtigung der eingebrachten Leistung z.B. über Kraftsensoren an den Pedalen oder Drehmomentsensoren an geeigneten Stellen kann aus allen Daten auf den aktuellen Windwiderstand zurückgeschlossen werden. Das hilft dem Fahrer eine optimale Position während der Fahrt einzunehmen und auch zu behalten, da die relevanten Werte periodisch neu ermittelt und angezeigt werden. Insbesondere erfolgt eine Berechnung mit einer Frequenz von wenigstens 5 mal pro Minute, vorzugsweise von wenigstens 20 mal pro Minute. Auch Frequenzen von 0,5 Hz oder 1 Hz oder 10 Hz oder mehr sind möglich.

Insgesamt werden eine vorteilhafte Zweiradkomponente und ein vorteilhaftes Verfahren offenbart, womit verbesserte Möglichkeiten zur Messung von Daten an einem Fahrrad ermöglicht werden. Je nach Lage eines Luftdrucksensors zur Ermittlung des absoluten Umgebungsdrucks wird das Ergebnis der Messung durch die Geschwindigkeit des Fahrrads, die Windgeschwindigkeit und die Windrichtung beeinflusst, und kann somit an sich zunächst ein ungenaues Ergebnis liefern. Werden zur Staudruckmessung beispielsweise Luftdrucksensoren mit einem Pitot-Röhrchen eingesetzt, welche nach vorn in Fahrtrichtung geöffnet sind, ergibt sich dort ein Totaldruck, der von dem absolut vorherrschenden Luftdruck in der Umgebung und von der Fahrgeschwindigkeit abhängt. Dieses Drucksignal alleine reicht zur Bestimmung einer Höhe oder Steigung nicht aus, da dann der Eindruck einer Steigung entstehen würde, wenn der Fahrer in der Ebene beschleunigt.

Befindet sich der Luftdrucksensor zur Ermittlung des absoluten Luftdrucks in der Umgebung hingegen beispielsweise in dem Gehäuse der Zweiradkomponente oder in der Messeinrichtung 2, so staut sich die Luft vor dem Gehäuse als Folge des Fahrtwindes bzw. der Fahrgeschwindigkeit und erzeugt an der vorderen Spitze an dem Gehäuse einen Totaldruck, der den im Inneren des Gehäuses gemessenen absoluten Luftdruck auch negativ (oder positiv) beeinflusst.

Wird der Luftdrucksensor zur Erfassung des absoluten Luftdrucks auf einer Seite des Gehäuses an einer Öffnung angeordnet, so wird das Ergebnis durch den Bernoulli-Effekt ebenfalls negativ beeinflusst. Dabei kann ein Unterdruck durch die vorbeistreichende Luft erzeugt werden, der den absoluten Druck auch wieder - geschwindigkeitsabhängig - negativ beeinflussen würde.

Deshalb ist eine Korrektur des Luftdrucksignals 21 durch den Staudruckwert 26 sinnvoll und vorteilhaft, wenn geringe und auch geringste Steigungen mit der Zweiradkomponente 1 ermittelt werden sollen. Die Korrektur erfolgt insbesondere zusammen mit einer Kalibrierungsmatrix, die bei vorherigen Versuchen unter bekannten Bedingungen aufgenommen wurde. Dabei werden insbesondere für Variationen der Relativgeschwindigkeit und/oder Variationen des Gierwinkels Kalibrierwerte erfasst und gespeichert. Eine Korrektur ist beispielsweise vorteilhaft und wichtig, um eine ausreichend genaue Ermittlung des Luftwiderstands durchzuführen.

Die Korrektur eines Höhenwertes 24 mittels eines Höhensignals 36 eines Satellitensensors ist vorteilhaft, da auch bei Rundfahrten die Zweiradkomponente am Ende der Rundfahrt die gleiche Höhe anzeigt wie zu Beginn der Rundfahrt.

Aufgrund der relativ großen Abstufung bei der Messung wird deshalb in der Regel ein Höhenprofil über Luftdrucksensoren erfasst. Bei bekannten Fahrradcomputern tritt es dann aber häufig auf, dass aufgrund von Luftdruckschwankungen die Höhenangaben zu Beginn und am Ende einer Rundfahrt unterschiedlich sind. Tatsächlich ist der Fahrer dabei aber eine komplette Runde gefahren und befindet sich am Ende auf exakt derselben Höhe wie zu Beginn der Tour.

Durch die hier offenbarte Kombination der Auswertung von Satellitensensoren und Drucksensoren kann eine sehr genaue Höhenbestimmung und insbesondere eine sehr genaue Bestimmung der Steigung eines Weges oder einer Strecke durchgeführt werden. Die zum Antrieb des Fahrrads benötigte Leistung hängt in erheblichem Maße von der Beschleunigung, der gegebenenfalls vorhandenen Steigung, dem Rollwiderstand und dem Luftwiderstand abhängt, kann hiermit eine hohe Genauigkeit erreicht werden.

Mit der Erfindung kann der Fahrer seine Sitzposition und auch seine Zweiradkomponenten und sonstige Ausrüstung wie den Helm, den Anzug, die Kleidung etc. mit vermessen und während der Fahrt beurteilen. Damit kann ein Fahrer die für ihn optimale Sitzposition und Kombination aus Zweiradteilen und Ausrüstung herausfinden und z. B. den für sich aerodynamisch besten Helm ermitteln, der bei seiner bevorzugten Position den geringsten Luftwiderstand bietet.

Neben den beschriebenen Möglichkeiten zur Kalibrierung des Luftdrucksensors bei der Fahrt ist es auch möglich, eine erneute Kalibrierung durchzuführen, wenn der Luftdrucksensor eine bestimmte Steigung oder ein bestimmtes Gefälle festgestellt hat.

Zum Beispiel nach einer Steigung oder einem Gefälle von 5 m oder 10 m. Möglich ist es auch, dass in bestimmten zeitlichen Abständen eine Rekalibrierung erfolgt. Möglich ist es auch, dass eine Kombination von zeitlicher Kalibrierung und Höhendifferenzüberschreitung durchgeführt wird.

Für die Messung von Luftdruckwerten werden vorzugsweise Luftdrucksensoren eingesetzt, die einen Messbereich aufweisen, der wenigstens 25% und insbesondere wenigstens 50% des Normaldrucks von (etwa) 100 kPa umfasst. Bevorzugt sind Luftdrucksensoren zur Erfassung des Umgebungsdrucks oder des Totaldrucks, die einen Messbereich größer 30 kPa und insbesondere wenigstens 50 kPa oder 60 kPa oder 80kPa aufweisen.

In bevorzugten Ausgestaltungen ist der Messbereich von den eingesetzten Differenzdrucksensoren kleiner als der von den eingesetzten Luftdrucksensoren. Differenzdrucksensoren werden insbesondere zur Erfassung des Staudruckes und/oder des Gierwinkels eingesetzt. Vorzugsweise ist ein Messbereich eines eingesetzten Differenzdrucksensors kleiner als 20 kPa und insbesondere kleiner als 10 kPa und besonders bevorzugt kleiner als 5 kPa oder 2 kPa oder 1 kPa. In einem konkreten Beispiel werden Differenzdrucksensoren eingesetzt, die einen Messbereich von 0,5 kPa (+/-20%) aufweisen. Dadurch wird eine hohe Auflösung und Genauigkeit ermöglicht.

Der Messbereich eines Luftdrucksensors zur Erfassung des Umgebungsdrucks oder des Totaldrucks ist vorzugsweise größer als der Messbereich eines Differenzdrucksensors für den Staudruck oder zur Bestimmung des Gierwinkels.

Ein Verhältnis eines Messbereichs eines Luftdrucksensors zur Erfassung des Umgebungsdrucks oder des Totaldrucks zu einem Messbereich eines Differenzdrucksensors für den Staudruck oder zur Bestimmung des Gierwinkels ist vorzugsweise größer als 5:1 und insbesondere größer 10:1 und besonders bevorzugt größer 50:1.

In allen Ausgestaltungen ist es bevorzugt, dass eine Temperaturkorrektur der Messwerte durchgeführt wird, um Temperatureinflüsse zu vermeiden.

Die Ausgestaltung der Messsonde bzw. des Sondenkörpers 5 ist vorteilhaft, da damit unabhängig von den äußeren Bedingungen ein Fahrrad betrieben werden kann. Durch die Ausgestaltung der Luftführung im Inneren des Sondenkörpers 5 wird es zuverlässig verhindert, dass eventuell ein dringendes Wasser zu einem Luftdrucksensor geleitet wird. Und falls einmal ein Tröpfchen Wasser oder Schmutz eingedrungen sein sollte, wird es in dem Aufnahmeraum 17 einer Kammer 15 zurückgehalten. Anschließend kann das Wasser dort beispielsweise durch Verdampfung wieder nach außen austreten oder nach Abnahme des insbesondere ansteckbaren Sondenkörpers 5 manuell gereinigt, durchgespült und/oder ausgeblasen werden. Durch die Luftkanäle und deren Abmessungen und die Kammer(n) wird eine Labyrinthdichtung mit einem zusätzlichen Aufnahmeraum zur Verfügung gestellt, sodass eine Wasserdichtigkeit der Messsonde 4 unter allen im alltäglichen Betrieb auftretenden Bedingungen erreicht wird.

Mit einer konventionellen Membrane im Inneren der Messsonde, um den Zufuhrkanal 11 mechanisch von dem Sensorkanal 12 abzutrennen, wird in der Regel eine ausreichende Dichtigkeit erzielt. Nachteilig ist aber, dass die Genauigkeit erheblich verringert wird und somit das Messergebnis verschlechtert wird, sodass eine ausreichend genaue Ermittlung eines Luftwiderstandsbeiwerts nicht erzielbar ist. Mit der hier offenbarten Messsonde 4 werden eine ausreichende Dichtigkeit und eine ausreichende Genauigkeit erreicht.

Vorzugsweise wird der Sondenkörper 5 durch einen 3D-Druckverfahren wenigstens teilweise oder vollständig aus Kunststoff und/oder wenigstens teilweise oder vollständig aus Metall hergestellt. In dem Inneren kann eine Dichtung bzw. Labyrinthdichtung integral ausgebildet ist. Ein 3D-Druckverfahren ermöglicht es, einen Sondenkörper viel leichter herzustellen als mit konventioneller Technologie. So kann dort, wo sonst aus verfahrenstechnischen Gründen volles Material sein muss, ein Hohlraum erzeugt werden.

### Bezugszeichenliste:

| | | | |
|---|---|---|---|
| 1 | Zweiradkomponente | 30b | Öffnung |
| 2 | Messeinrichtung | 30c | Differenzdrucksensor |
| 3 | Gehäuse | 32 | Gierwinkel |
| 4 | Messsonde | 33 | Relative Windrichtung und |
| 5 | Sondenkörper | | Windstärke |
| 6 | Öffnung in 5 | 34 | Fahrgeschwindigkeit |
| 6a | Öffnung | 35 | Satellitensensor |
| 7 | Öffnung | 36 | Höhensignal |
| 8 | Abmessung von 5 | 37 | Feuchtesensor |
| 10 | Luftführung | 38 | Beschleunigungssensor |
| 11 | Luftkanal, Zufuhrkanal | 40 | Steuereinrichtung |
| 12 | Luftkanal, Sensorkanal | 41 | erster Messverlauf |
| 13 | Luftkanal, Zwischenkanal | 42 | zweiter Messverlauf |
| 14 | Labyrinthdichtung | 43 | dritter Messverlauf |
| 15 | Kammer | 44 | Steigungsverlauf |
| 15a | Teilkammer | 50 | Recheneinrichtung |
| 15b | Teilkammer | 51 | Speicher |
| 15c | Zwischenwand | 52 | Datenschnittstelle, |
| 15d | Verbindungsöffnung | | Netzwerkschnittstelle |
| 16 | Kammer | 53 | Kalibrierdaten |
| 17 | Aufnahmeraum in 15, 16 | 54 | Energiequelle |
| 18 | Wasser | 100 | Fahrrad |
| 19 | Querschnitt von 11-13 | 101 | Rad, Vorderrad |
| 20 | Luftdrucksensor, | 102 | Rad, Hinterrad |
| | Absolutdrucksensor | 103 | Rahmen |
| 21 | Luftdrucksignal von 20, | 104 | Gabel, Federgabel |
| | Sensorwert von 20, | 106 | Lenker |
| 22 | korrigierter | 107 | Sattel |
| | Umgebungsdruckwert | 109 | Speiche |
| 23 | Höhenmaß | 110 | Felge |
| 24 | Höhenwert | 112 | Tretkurbel |
| 25 | Staudrucksensorsystem, | 115 | Geschwindigkeitssensor |
| | Pitot-Sensor | 116 | Leistungssensor, |
| 25c | Differenzdrucksensor | | Kraftsensor |
| 26 | Staudruckwert, | 120 | Höhe |
| | Sensorwert von 25 | 200 | Weg |
| 27 | Höhenänderung | 201 | Steigungswert, |
| 28 | Referenzsignal | | Steigungswinkel |
| 29 | Störsignal | 202 | Höhenverlauf |
| 30 | Gier-Sensorsystem | 300 | Satellitensystem |
| 30a | Öffnung | 301 | Satellit |

## Patentansprüche

1. Zweiradkomponente (1) mit einer Messeinrichtung (2) mit einem Gehäuse (3) und wenigstens einer damit verbundenen Messsonde (4), umfassend wenigstens einen ungeheizten Sondenkörper (5), der wenigstens eine äußere Öffnung (6, 7) aufweist, wobei die äußere Öffnung (6) durch eine Luftführung (10) mit wenigstens einem von der äußeren Öffnung (6) entfernt angeordneten Luftdrucksensor (20, 25, 30) verbunden ist;
**dadurch gekennzeichnet dass**
die Luftführung (10) wenigstens zwei Luftkanäle (11, 12) und wenigstens eine innere Kammer (13) umfasst, die mit wenigstens zwei Luftkanälen (11, 12) verbunden ist, wobei einer der Luftkanäle (11, 12) als Zufuhrkanal (11) ausgebildet ist und an der äußeren Öffnung (5) beginnt und wobei ein anderer Luftkanal (12) als Sensorkanal (12) dient und die innere Kammer (15) mit dem Luftdrucksensor (20, 25, 30) verbindet und wobei wenigstens einer der Luftkanäle einen minimalen lichten Durchmesser kleiner 2,5 mm aufweist.

2. Zweiradkomponente (1) nach dem vorhergehenden Anspruch, wobei wenigstens einer der Luftkanäle an der äußeren Öffnung einen Durchmesser kleiner 2 mm aufweist.

3. Zweiradkomponente (1) nach Anspruch 1 oder 2, wobei an der inneren Kammer (15) wenigstens ein Aufnahmeraum (17) für eventuell eingedrungenes Wasser (18) ausgebildet ist.

4. Zweiradkomponente (1) nach dem vorhergehenden Anspruch, wobei wenigstens ein Aufnahmeraum (18) topfförmig ausgebildet ist.

5. Zweiradkomponente (1) nach einem der vorhergehenden Ansprüche, wobei die innere Kammer (15) wenigstens eine Zwischenwand (15c) aufweist, welche die innere Kammer (15) in wenigstens zwei Teilkammern (15a, 15b) unterteilt und wobei die zwei Teilkammern (15a, 15b) über wenigstens eine Verbindungsöffnung (15d) miteinander verbunden sind und wobei im bestimmungsgemäßen Gebrauch die Verbindungsöffnung (15d) vom unteren Ende der inneren Kammer (15) beabstandet angeordnet ist.

6. Zweiradkomponente (1) nach einem der vorhergehenden Ansprüche, wobei an der Luftführung (10) wenigstens eine Art von Labyrinthdichtung ausgebildet ist.

7. Zweiradkomponente (1) nach einem der vorhergehenden Ansprüche, wobei die Labyrinthdichtung (14) zwei oder mehr innere Kammern (15, 16) und dazwischen angeordnete Luftkanäle (13) umfasst.

8. Zweiradkomponente (1) nach einem der vorhergehenden Ansprüche, wobei das Gehäuse (3) und/oder der Sondenkörper (5) aerodynamisch ausgebildet ist und wobei der Sondenkörper (5) langgestreckt und mit einer im Wesentlichen rotationssymmetrischen Außenfläche ausgebildet ist.

9. Zweiradkomponente (1) nach einem der vorhergehenden Ansprüche, wobei wenigstens ein wesentlicher Teil des Sondenkörpers (5) unter Ausbildung der Luftführung (10) durch ein 3D-Druckverfahren hergestellt ist.

10. Zweiradkomponente (1) nach einem der vorhergehenden Ansprüche, wobei der Sondenkörper (5) wenigstens zwei oder drei Öffnungen (6, 7) aufweist, denen wenigstens ein Luftdrucksensor (20, 25, 30) zugeordnet sind.

11. Zweiradkomponente (1) nach einem der vorhergehenden Ansprüche, wobei der Sondenkörper (5) an das Gehäuse (3) angesteckt ist und/oder wobei der Sondenkörper (5) wenigstens zu einem erheblichen Teil aus wenigstens einem Metall besteht.

12. Zweiradkomponente (1) nach einem der vorhergehenden Ansprüche, wobei der wenigstens eine Luftdrucksensor (20, 25, 30) in dem Gehäuse (3) angeordnet ist.

13. Zweiradkomponente (1) nach einem der vorhergehenden Ansprüche, wobei in dem Gehäuse (3) eine Recheneinrichtung (50) angeordnet ist.

14. Zweiradkomponente(1) nach einem der vorhergehenden Ansprüche, wobei die Abmessungen (8) der äußeren Öffnungen (6, 7) und der inneren Luftführung (10) so ausgelegt sind, dass der resultierende Querschnitt (19) der Luftkanäle (11-13) klein genug ist, dass die Wasseroberflächenspannung an dem Querschnitt (19) des Luftkanals (11-13)der Kraft widersteht, die sich aus dem dynamischen Druck der Luft bei Luftgeschwindigkeiten von bis zu 35 m/s ergibt, und dadurch das Eindringen von Wasser (18) bis zu dem Luftdrucksensor (20, 25, 30) verhindert.

15. Zweiradkomponente (1) nach einem der vorhergehenden Ansprüche, wobei jede Innenkammer (15, 16) einen Aufnahmeraum (17) enthält, in dem sich Wasser (18), das durch die äußeren Öffnungen (6) in die Messsonde (4) eintritt, sammeln kann und so positioniert ist, dass es vom Ausgang der inneren Kammer (15) in Richtung des Luftdrucksensors (20, 25, 30) entfernt ist.

## Claims

1. Bicycle component (1) with a measuring device (2) with a housing (3) and at least one measuring probe (4) connected therewith, comprising at least one unheated probe body (5) having at least one exterior opening (6, 7), the exterior opening (6) being connected through an air guide (10) with at least one barometric pressure sensor (20, 25, 30) disposed remote from the exterior opening (6),
**characterized in that** the air guide (10) comprises at least two air ducts (11, 12) and at least one internal chamber (13) connected with at least two air ducts (11, 12), wherein one of the air ducts (11, 12) is configured as a supply duct (11) and begins at the exterior opening (5), and wherein another air duct (12) serves as a sensor duct (12) and connects the internal chamber (15) with the barometric pressure sensor (20, 25, 30), and wherein at least one of the air ducts has a minimum clear diameter of less than 2.5 mm.

2. The bicycle component (1) according to the preceding claim,
wherein at least one of the air ducts has a diameter of less than 2 mm at the exterior opening.

3. The bicycle component (1) according to claim 1 or 2, wherein the internal chamber (15) is configured with at least one takeup space (17) for any penetrated water (18).

4. The bicycle component (1) according to the preceding claim,
wherein at least one takeup space (18) is configured pot-shaped.

5. The bicycle component (1) according to any of the preceding claims, wherein the internal chamber (15) comprises at least one partition wall (15c) which subdivides the internal chamber (15) into at least two chamber sections (15a, 15b), and wherein the two chamber sections (15a, 15b) are interconnected through at least one connecting opening (15d), and wherein in use as intended the connecting opening (15d) is disposed spaced apart from the bottom end of the internal chamber (15).

6. The bicycle component (1) according to any of the preceding claims, wherein the air guide (10) is configured with at least one type of labyrinth seal.

7. The bicycle component (1) according to any of the preceding claims, wherein the labyrinth seal (14) comprises two or more internal chambers (15, 16) and disposed in-between, air ducts (13) .

8. The bicycle component (1) according to any of the preceding claims, wherein the housing (3) and/or the probe body (5) is configured aerodynamically, and wherein the probe body (5) is configured elongated and has a substantially rotationally symmetrical outer surface.

9. The bicycle component (1) according to any of the preceding claims, wherein at least a substantial part of the probe body (5) is manufactured by way of 3D printing, forming the air guide (10) .

10. The bicycle component (1) according to any of the preceding claims, wherein the probe body (5) comprises at least two or three openings (6, 7), to which at least one barometric pressure sensor (20, 25, 30) is assigned.

11. The bicycle component (1) according to any of the preceding claims, wherein the probe body (5) is plugged onto the housing (3), and/or wherein the probe body (5) consists at least considerably of at least one metal.

12. The bicycle component (1) according to any of the preceding claims, wherein the at least one barometric pressure sensor (20, 25, 30) is disposed in the housing (3).

13. The bicycle component (1) according to any of the preceding claims, wherein a computer (50) is disposed in the housing (3).

14. The bicycle component (1) according to any of the preceding claims, wherein the dimensions (8) of the exterior openings (6, 7) and of the internal air guide (10) are designed such that the resulting cross section (19) of the air ducts (11 - 13) is small enough for the water surface tension at the cross section (19) of the air duct (11 - 13) to withstand the force ensuing from the dynamic air pressure with air speeds of up to 35 m/s, and thus to prevent water (18) from penetrating up to the barometric pressure sensor (20, 25, 30).

15. The bicycle component (1) according to any of the preceding claims, wherein each internal chamber (15, 16) contains a takeup space (15), in which water (18) entering into the measuring probe (4) through the exterior openings (6) may collect, and is positioned such that it is remote from the outlet of the internal chamber (15) in the direction of the barometric pressure sensor (20, 25, 30).

## Revendications

1. Élément de deux roues (1) doté d'un appareil de mesure (2) doté d'un boîtier (3) et d'au moins une sonde de mesure (4) qui y est reliée, comprenant au moins un corps de sonde (5) non chauffé qui présente au moins une ouverture (6, 7) extérieure, ladite ouverture extérieure (6) étant reliée par une conduite d'air (10) à au moins un capteur de pression atmosphérique (20, 25, 30) agencé à distance de ladite ouverture extérieure (6) ;
**caractérisé en ce que** ladite conduite d'air (10) comprend au moins deux conduits d'air (11, 12) et au moins une chambre interne (13) qui est reliée à au moins deux conduits d'air (11, 12), un desdits conduits d'air (11, 12) étant conçu comme conduit d'alimentation (11) et partant de l'ouverture extérieure (5), et un autre conduit d'air (12) servant de conduit capteur (12) et reliant la chambre interne (15) au capteur de pression atmosphérique (20, 25, 30) et au moins un desdits conduits d'air présentant un diamètre libre minimum de moins de 2,5 mm.

2. Élément de deux roues (1) selon la revendication précédente, au moins un des conduits d'air présentant à l'ouverture extérieure un diamètre de moins de 2 mm.

3. Élément de deux roues (1) selon la revendication 1 ou 2, au moins une cellule de réception (17) étant conçue dans la chambre interne (15) pour de l'eau (18) qui aurait éventuellement pénétrée à l'intérieur.

4. Élément de deux roues (1) selon la revendication précédente, au moins une cellule de réception (18) étant conçue sous forme de pot.

5. Élément de deux roues (1) selon l'une quelconque des revendications précédentes, ladite chambre interne (15) présentant au moins une cloison intermédiaire (15c) qui divise la chambre interne (15) en au moins deux chambres partielles (15a, 15b) et lesdites deux chambres partielles (15a, 15b) étant reliées l'une à l'autre par au moins une ouverture de liaison (15d) et ladite ouverture de liaison (15d) étant, dans une utilisation correcte, agencée à distance de l'extrémité inférieure de la chambre interne (15) .

6. Élément de deux roues (1) selon l'une quelconque des revendications précédentes, au moins une sorte de joint d'étanchéité à labyrinthe étant conçue au niveau de la conduite d'air (10).

7. Élément de deux roues (1) selon l'une quelconque des revendications précédentes, ledit joint d'étanchéité à labyrinthe (14) comprenant deux ou plus chambres internes (15, 16) et des conduits d'air (13) agencés entre celles-ci.

8. Élément de deux roues (1) selon l'une quelconque des revendications précédentes, le boîtier (3) et/ou le corps de sonde (5) étant conçu aérodynamique et le corps de sonde (5) étant allongé et conçu avec une surface extérieure sensiblement symétrique en rotation.

9. Élément de deux roues (1) selon l'une quelconque des revendications précédentes, au moins une partie essentielle du corps de sonde (5) étant construit par formation de la conduite d'air (10) par processus d'impression 3d.

10. Élément de deux roues (1) selon l'une quelconque des revendications précédentes, ledit corps de sonde (5) présentant au moins deux ou trois ouvertures (6, 7) auxquelles sont au moins associés un capteur de pression atmosphérique (20, 25, 30).

11. Élément de deux roues (1) selon l'une quelconque des revendications précédentes, ledit corps de sonde (5) étant connecté au boîtier (3) et/ou ledit corps de sonde (5) se composant, au moins pour une partie essentielle, d'au moins un métal.

12. Élément de deux roues (1) selon l'une quelconque des revendications précédentes, ledit au moins un capteur de pression atmosphérique (20, 25, 30) étant agencé dans le boîtier (3).

13. Élément de deux roues (1) selon l'une quelconque des revendications précédentes, au moins un dispositif de calcul (50) étant agencé dans ledit boîtier (3).

14. Élément de deux roues (1) selon l'une quelconque des revendications précédentes, les dimensions (8) des ouvertures extérieures (6, 7) et du conduit d'air intérieur (10) étant configurées de sorte que la section droite (19) des conduits d'air (11 à 13) qui en résulte est assez petite pour que la tension superficielle de l'eau au niveau de la section droite (19) du conduit d'air (11 à 13) résiste à la force qui résulte de la pression dynamique de l'air à des vitesses d'air de jusqu'à 35 m/s, empêchant ainsi que de l'eau (18) pénètre jusqu'au capteur de pression atmosphérique (20, 25, 30) .

15. Élément de deux roues (1) selon l'une quelconque des revendications précédentes, chaque chambre intérieure (15, 16) contenant une cellule de réception (17) dans laquelle de l'eau (18) qui pénètre dans la sonde de mesure (4) par les ouvertures extérieures (6), peut s'accumuler et est positionnée de sorte qu'elle est à distance de la sortie de la chambre interne (15) en direction du capteur de pression atmosphérique (20, 25, 30).
